# FASCICULE DE BREVET EUROPEEN

(11) **EP 0 770 392 B1**
(45) Date de publication et mention de la délivrance du brevet: **03.08.2005**
(21) Numéro de dépôt: 96402182.8
(22) Date de dépôt: 14.10.1996
(51) Int. Cl.: A61K 33/30, A61K 33/32, A61K 33/24, A61K 33/00, A61P 17/00, A61P 25/00, A61P 9/00, A61P 37/00, A61P 13/00, A61P 1/00, A61K 7/48

(54) **Utilisation de sels de manganèse, d'yttrium ou de certains lanthanides pour la préparation d'une composition cosmétique, dermatologique ou pharmaceutique destinée surtout à traiter les peaux sensibles**
Verwendung von Mangan-, Yttrium- oder einigen Lanthanidsalzen in einer kosmetischen, dermatologischen oder pharmazeutischen Zusammensetzung insbesondere zur Behandlung empflindlicher Haut
Use of manganese, yttrium or certain lanthanides salts in a cosmetic, dermatological or pharmaceutical composition to treat in particular sensitive skins

(30) Priorité: 26.10.1995 FR 9512658
(43) Date de publication de la demande: 02.05.1997
(73) Titulaire: L'OREAL, 75008 Paris (FR)
(72) Inventeur: Breton, Lionel, 78000 Versailles (FR); de Lacharriere, Olivier, 75015 Paris (FR)
(74) Mandataire: Lhoste, Catherine

(56) Documents cités:
- EP-A- 0 085 579
- EP-A- 0 135 312
- EP-A- 0 217 975
- EP-A- 0 250 300
- EP-A- 0 409 662
- WO-A-94/02130
- WO-A-96/19182
- WO-A-96/19183
- DE-A- 2 715 711
- DE-A- 3 443 985
- FR-M- 5 394
- GB-A- 2 217 602
- US-A- 4 938 969
- US-A- 5 401 730
- DATABASE WPI Week 199612, Derwent Publications Ltd., London, GB; Class A96, AN 1996-114522 & RU 2 038 072 A (ORG CHEM TECHN RES INST) 27 Juin 1995

## Description

La présente invention se rapporte à l'utilisation d'au moins un sel de lanthanide, de manganèse ou d'yttrium dans une composition cosmétique, dermatologique ou pharmaceutique comme antagoniste de substance P, pour la préparation d'une composition pharmaceutique, dermatologique ou cosmétique, destinée à traiter les désordres associés à un excès de synthèse et/ou de libération de substance P et plus spécialement les peaux sensibles.

Il existe chez les mammifères des polypeptides appartenant à la famille des tachykinines qui induisent sur les fibres musculaires lisses des contractions rapides. Parmi les composés de cette famille on peut citer la neurokinine β, neurokinine α et la substance P.

La substance P est un élément chimique polypeptidique (undécapeptide), élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P ; il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des systèmes nerveux central et périphérique.

De par la distribution ubiquitaire de la substance P, de très nombreux désordres sont associés à un excès de synthèse et/ou de libération de substance P.

La substance P intervient notamment dans la transmission de la douleur (dentaire, cutanée, tympanique) et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie, maladies psychotiques), dans des maladies respiratoires (telles que par exemple les broncho-pneumonie, toux, emphysème, bronchiolite) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn, gastrites, gastro-entérites, spasticites intestinales), dans des désordres cutanés (tels que par exemple le psoriasis, les maladies prurigineuses, l'herpès, les photodermatoses, les dermatites atopiques, les dermatites de contact, les lichens, les prurigos, les prurits, les rosacées, les ulcères, le zona, les démodécies, la couperose, les peaux sensibles, les dartres, les érythèmes solaires et émotionnels, les piqûres d'insectes), dans des fibroses et autres troubles de la maturation des collagènes (tels que par exemple la sclérodermie), dans des troubles cardio-vasculaires, des troubles vasospastiques (tels que par exemple les migraines, la maladie de Raynaud), dans des désordres immunologiques, dans des troubles du tractus urinaire et ou génital (tels que par exemple l'incontinence, la cystite), dans des maladies rhumatismales dans certaines maladies dermatologiques (telles que l'eczéma) et dans les affections ophtalmologiques (telles que par exemple les conjonctivites, les uvéites, les prurits oculaires, les blépharites, les douleurs oculaires et les irritations).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P. Particulièrement, pour qu'une substance soit reconnue comme un antagoniste de substance P, elle doit induire une réponse pharmacologique cohérente (incluant ou non sa fixation au récepteur de la substance P) notamment dans l'un des tests suivants :
- la substance antagoniste doit diminuer l'extravasation du plasma au travers de la paroi vasculaire induite par la capsaïcine ou par une stimulation nerveuse antidromique,
ou bien
- la substance antagoniste doit provoquer une inhibition de la contraction des muscles lisses induites par l'administration de substance P.

A ce jour, des antagonistes de substance P sont utilisés pour traiter les désordres indiqués ci-dessus et sont cités dans de nombreux documents.

Les demandes de brevet WO 96/19182 et WO 96/19183 décrivent respectivement l'utilisation de cations divalent de manganèse et leurs sels ou de cations trivalents de lanthanide et leurs sels, et de sels d'aluminium et d'étain en tant qu'agent pour le traitement topique des peaux irritées, ainsi que des composition les comprenant avec optionellement un agent irritant.

Cependant aucun de ces documents n'envisage, ni ne suggère qu'un sel de lanthanide, de manganèse, d'yttrium puisse avoir une activité antagoniste de substance P telle que définie ci-dessus et donc puisse être notamment utilisé pour traiter les désordres indiqués ci-dessus.

La demanderesse a découvert qu'un sel choisi parmi certains sels de lanthanide, de manganèse ou d'yttrium répond aux caractéristiques définies comme caractérisant un antagoniste de substance P et peut donc être utilisé comme antagoniste de substance P.

Ainsi, l'invention concerne l'utilisation d'au moins un sel choisi parmi certains sels de lanthanide, de manganèse, d'yttrium et leur mélange comme antagoniste de substance P dans une composition cosmétique, dermatologique ou pharmaceutique.

L'invention concerne également l'utilisation d'au moins un sel choisi parmi certains sels de lanthanide, de manganèse, d'yttrium et leur mélange pour la préparation d'une composition pharmaceutique, dermatologique ou pharmaceutique destinée à traiter les désordres associées à un excès de synthèse et/ou de libération de substance P.

Par lanthanide, on entend les éléments de numéro atomique z allant de 57 à 71, c'est-à-dire le lanthane, le cérium, le praséodyme, le néodyme, le prométhium, le samarium, l'europium, le gadolinium, le terbium, le dysprosium, l'holmium, l'erbium, le thulium, l'ytterbium, le lutétium.

L'invention s'applique à tous les désordres liés à un excès de synthèse et/ou de libération de substance P cités précédemment.

Ainsi, selon un aspect particulier, l'invention a pour objet l'utilisation d'au moins un sel de lanthane, de cérium, de praséodyme, de prométhium, de samarium, d'europium, de gadolinium, de terbium, de dysprosium, d'holmium, d'erbium, de thulium, d'ytterbium, de lutétium, de manganèse ou d'yttrium dans une composition cosmétique, dermatologique ou pour la préparation d'une composition pharmaceutique destinée à traiter les désordres du système nerveux central, les fibroses, les troubles de la maturation du collagène, les troubles cardio-vasculaires, les troubles vasospastiques, les désordres immunologiques ainsi que les troubles du tractus urinairogénital et les troubles pancréatiques.

Dans le domaine des désordres cutanés, il est connu que certaines peaux sont plus sensibles que d'autres. Toutefois les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.

Des tests ont été mis au point pour cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques, dermatologiques ou des savons. En général, ces signes sont associés à une peau hyperséborrhéique ou acnéique, voire rosaéciforme avec ou sans dartres, et à un érythème.

Les cuirs chevelus "sensibles" ont une sémiologie clinique plus univoque : les sensations de prurit et/ou de picotements et/ou d'échauffements sont essentiellement déclenchées par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments ou des produits cosmétiques ou dermatologiques. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Les signes cliniques de la peau sensible sont essentiellement subjectifs: picotements, fourmillements, prurits, tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Ainsi la demanderesse a découvert que l'une des caractéristiques essentielles des peaux sensibles est liée à la libération et/ou synthèse de substance P et donc que l'utilisation d'antagonistes de substance P peut permettre d'obtenir un effet préventif et/ou curatif des peaux sensibles.

Pour traiter les peaux sensibles, la demanderesse a donc envisagé d'utiliser des antagonistes de substance P. Elle a en effet constaté de manière surprenante que l'incorporation d'un antagoniste de substance P dans une composition destinée à un usage topique permettait d'éviter l'irritation et/ou les sensations dysesthésiques et/ou les prurits de la peau et/ou des muqueuses.

Aussi l'invention se rapporte donc plus particulièrement à l'utilisation d'au moins un sel choisi parmi les sels de néodyme, de prométhium, de samarium, d'europium, de terbium, de dysprosium, d'holmium, d'erbium, de thulium, d'ytterbium ou d'yttrium et leur mélange dans une composition cosmétique, dermatologique ou pharmaceutique ou pour la préparation d'une composition pharmaceutique, cosmétique ou dermatologique, destinée à traiter les peaux sensibles.

Aussi la présente invention se rapporte à l'utilisation d'au moins un sel choisi parmi les sels de de néodyme, de prométhium, de samarium, d'europium, de terbium, de dysprosium, d'holmium, d'erbium, de thulium, d'ytterbium ou d'yttrium et leur mélange dans une composition cosmétique, dermatologique dans ou pour la préparation d'une composition pharmaceutique, destinée à prévenir et/ou à lutter contre les irritations cutanées et/ou les dartres et/ou les érythèmes et/ou les sensations d'échauffement et/ou de dysesthésie et/ou les prurits de la peau et/ou les muqueuses.

Les sels utilisés peuvent être par exemple des carbonates, des bicarbonates, des sulfates, des glycérophosphates, des borates, des chlorures, des nitrates, des acétates, des hydroxydes, des persulfates ainsi que des sels d'α-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate).

De préférence, on utilise des chlorures et des nitrates avantageusement de néodyme de gadolinium ou d'yttrium.

Selon l'invention, le sel peut être utilisé en une quantité allant de 10⁻⁵ % à 20 % du poids total de la composition et préférentiellement en une quantité allant de 10⁻² % à 15 % du poids total de la composition et mieux de 0,5 à 8% du poids total de la composition.

Le sel peut être utilisé dans une composition qui doit être ingérée, injectée ou appliquée sur la peau (sur toute zone cutanée du corps), les cheveux, les ongles ou les muqueuses (buccale, jugale, gingivale, génitale, anale, nasale, conjonctive). Selon le mode d'administration, cette composition peut se présenter sous toutes les formes galéniques normalement utilisées.

Pour une application topique sur la peau, la composition peut avoir la forme notamment de solution aqueuse hydroalcoolique ou huileuse ou de suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème, ou de gel aqueux ou anhydres, de microémulsions ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, ou de mousses.

Pour l'injection, la composition peut se présenter sous forme de lotion aqueuse, de suspension huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits après-solaires, des lotions, gels ou mousses pour le soin de la peau, comme des lotions de nettoyage, des lotions après-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères et celles énoncées précédemment.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

Le sel utilisé selon l'invention peut aussi être incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, des shampooings antiparasitaires.

Les compositions peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales, (huile de vaseline), les huiles végétales (huile de soja, huile de palme, fraction liquide de beurre de karité, huile de toumesolfraction liquide du beurre de karité, huile de tournesol), les huiles animales (perhydrosqualène), les huiles de synthèse (huile de Purcellin), les huiles ou cires siliconées (cyclométhicone, diméthicone) et les huiles fluorées (perfluoropolyéthers), les cires d'abeille, de camauba ou paraffine. On peut ajouter à ces huiles des alcools gras (alcool cétylique), des acides gras (acide stéarique), des cires (cires d'abeilles, de carnauba, et de la paraffine).

Comme émulsionnants utilisables dans l'invention, on peut citer par exemple le stéarate de glycérol, le PEG-100 stearate, le polyglyceryl-3 hydroxylaurylether, le polysorbate 60.polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, l'éthylcellulose, le polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux ou bactériens et l'amidon.

Comme actifs lipophiles, on peut utiliser le rétinol (vitamine A) et ses dérivés, le tocophérol (vitamine E) et ses dérivés, les acides gras essentiels, les céramides, les huiles essentielles.

Selon l'invention on peut, entre autres, associer aux sels de lanthanide, de manganèse ou d'yttrium d'autres agents actifs destinés notamment à la prévention et/ou au traitement des affections cutanées.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et béta-hydroxy-carboxyliques ou -cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les antiseptiques ;
- les antimétabolites ;
- les agents pour lutter contre la chute des cheveux comme le minoxidil.

Ainsi, selon un mode particulier, l'invention se rapporte à l'utilisation d'au moins un sel choisi parmi les sels de lanthanide, de manganèse ou d'yttrium et leur mélange dans une composition comprenant au moins un agent choisi parmi les agents antibactériens, antiparasitaires, antifongiques, antiviraux, anti-inflammatoires, antiprurigineux, anesthésiques, kératolytiques, anti-radicaux libres, antiséborrhéiques, antipelliculaires, antiacnéiques et/ou les agents modulant la différenciation et/ou la prolifération et/ou la pigmentation cutanée.

De façon avantageuse, selon l'invention au moins un sel de lanthanide, de manganèse ou d'yttrium peut être associé à des produits à effet irritant utilisés couramment dans le domaine cosmétique, dermatologique ou pharmaceutique, produits qui sont parfois des actifs cosmétiques, dermatologiques ou pharmaceutiques. La présence d'un antagoniste de substance P sous la forme d'au moins un sel de lanthanide, de manganèse ou d'yttrium dans ou pour la fabrication d'une composition cosmétique, dermatologique ou pharmaceutique comprenant un produit ayant un effet irritant permet d'atténuer fortement, voire de supprimer cet effet irritant. Cela permet en outre d'augmenter la quantité d'actif à effet irritant par rapport à la quantité d'actif normalement utilisée, en vue d'une efficacité améliorée.

Ainsi, l'invention concerne également une composition cosmétique, dermatologique ou pharmaceutique comprenant dans un milieu cosmétiquement, dermatologiquement ou pharmaceutiquement acceptable au moins un produit à effet irritant, caractérisée en ce qu'elle contient en outre au moins un sel choisi parmi les sels de néodyme ou d'yttrium.

Comme produits à effet irritant, on peut citer par exemple les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxy-acides (l'acide salicylique et ses dérivés), les α-céto-acides, les β-céto-acides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

L'emploi d'antagoniste de substance P permet notamment de multiplier par 2 à 10 fois la quantité d'actif à effet irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, en diminuant notablement leur caractère irritant.

Bien évidemment, les compositions selon l'invention comprenant dans un milieu cosmétiquement, dermatologiquement ou pharmaceutiquement acceptable au moins un produit à effet irritant et au moins un sel choisi parmi les sels d'yttrium et de néodyme et leur mélange peuvent se présenter sous toute forme galénique connue, comme en particulier celles décrites précédement dans le texte.

On peut par ailleurs associer aux sels de l'invention des antagonistes de CGRP (Calcitonin Gene Related Peptide ou peptide lié au gène de la calcitonine) tels que les extraits d'lridacée comme l'Iris Pallida, des antagonistes de substance P tels que décrits dans la demande de brevet N° 94 05537 déposée par la demanderesse ou des sels de strontium, et des antagonistes d'histamine, d'interleukine-1 et/ou de TNF α (Tumor Necrosis Factor -alpha).

L'invention peut être mise en oeuvre notamment en appliquant les compositions hygiéniques, cosmétiques ou pharmaceutiques telles que définies ci-dessus selon la technique d'utilisation habituelle de ces compositions. Par exemple : application de crèmes, de gels, de sérums, de lotions, de laits de démaquillage ou de compositions après-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention.

Dans les compositions les proportions indiquées sont des pourcentages en poids.

| Crème de nettoyage (Formule A) | |
|---|---|
| Carbonate d'yttrium | 5,00 |
| Alcool cétylique | 2,00 |
| Stéarate de glycérol | 2,00 |
| Acide Stéarique | 2,00 |
| Polyglyceryl-3 hydroxylauryl Ether | 5,00 |
| Huile minérale Codex | 12,00 |
| Carbomer | 0,35 |
| Hydroxyde de sodium | 0,15 |
| Parfum qsp | |
| Méthyl paraben | 0,20 |
| Eau déminéralisée stérile qsp | 100,00 |

| Lait de nettoyage (Formule B) | |
|---|---|
| Nitrate de lanthane | 0,5 |
| Carbomer | 0,40 |
| Hydroxyde de sSodium | 0,10 |
| Huile minérale Codex | 5,00 |
| Stéarate de glycérol | 1,00 |
| Alcool cétylique | 0,50 |
| PEG 100 stéarate | 0,80 |
| Méthyl paraben | 0,20 |
| Parfum qsp | |
| Eau déminéralisée stérile qsp | 100,00 |

| Lotion de soin (Formule C) | |
|---|---|
| Chlorure de manganèse | 15,00 |
| Glycérol | 2,00 |
| Méthyl paraben | 0,15 |
| Parfum qsp | |
| Eau déminéralisée stérile qsp | 100,00 |

| Crème de soin (Formule D) | |
|---|---|
| Chlorure d'europium | 1,00 |
| Stéarate de glycérol | 1,00 |
| PEG 100 stéarate | 1,00 |
| Acide stéarique | 1,00 |
| Alcool cétylique | 2,00 |
| Huile de soja | 3,00 |
| Huile de palme | 2,00 |
| Cyclométhicone | 2,00 |
| Diméthicone | 1,00 |
| Polyacrylamide | 0,20 |
| Glycérol | 3,00 |
| Méthyl paraben | 0,20 |
| Parfum qsp | |
| Eau stérile déminéralisée qsp | 100,00 |

| Lotion démaquillante pour le visage (Formule E) | |
|---|---|
| Citrate de néodyme | 0,5 |
| Antioxydant | 0,05 |
| Isopropanol | 40,00 |
| Conservateur | 0,30 |
| Eau qsp | 100 % |

| Gel pour le soin du visage (Formule F) | |
|---|---|
| Chlorure d'holmium | 0,5 |
| Hydroxypropylcellulose (Klucel H vendu par la société) | 1,00 |
| Antioxydant | 0,05 |
| Isopropanol | 40,00 |
| Conservateur | 0,30 |
| Eau qsp | 100 % |

| Crème de soin de l'érythème solaire (émulsion huile-dans-eau) (Formule G) | |
|---|---|
| Chlorure de néodyme | 5,00 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de toumesol | 10,00 |
| Antioxydant | 0,05 |
| Parfum | 0,5 |
| Conservateur | 0,30 |
| Eau qsp | 100 % |

Exemples de formulations illustrant l'invention et particulièrement les compositions selon l'invention associant au moins sel de lanthanide, de manganèse ou de d'yttrium et un produit à effet irritant. Ces compositions ont été obtenues par simple mélange des différents composants.

| Composition 1 : Gel pour le traitement de l'acné | |
|---|---|
| Borate de manganèse | 5,00 |
| Acide tout trans rétinoïque | 0,05 |
| Hydroxypropylcellulose (klucel H) | 1,00 |
| Antioxydant | 0,05 |
| Isopropanol | 40,00 |
| Conservateur | 0,30 |
| Eau qsp | 100 % |

| Composition 2 : Lotion pour éliminer les cicatrices dues à l'acné | |
|---|---|
| ChlorureGlycérophosphate de néodymelithium | 1,5 |
| Acide glycolique | 50,00 |
| Hydroxypropylcellulose (Klucel H) | 0,05 |
| NaOH qsp | pH =2,80 |
| Ethanol qsp | 100 % |
| Conservateur | 0,30 |

## Revendications

1. Utilisation d'au moins un sel choisi parmi les sels d'yttrium, de lanthane, de cérium, de praséodyme, de prométhium, de samarium, d'europium, de gadolinium, de terbium, de dysprosium, d'holmium, d'erbium, de thulium, d'ytterbium, de lutétium, de manganèse et leur mélange, pour la fabrication d'une composition cosmétique, dermatologique ou pharmaceutique, destinée à traiter les désordres du système nerveux central, les fibroses, les troubles de la maturation du collagène, les troubles cardio-vasculaires, les troubles vasospastiques, les désordres immunologiques et/ou les troubles du tractus urogénital, les troubles pancréatiques.

2. Utilisation d'au moins un sel choisi parmi les sels d'yttrium, de néodyme, de prométhium, de samarium, d'europium, de terbium, de dysprosium, d'holmium, d'erbium, de thulium, d'ytterbium et leur mélange, pour la fabrication d'une composition cosmétique ou dermatologique, destinée à traiter les peaux sensibles.

3. Utilisation selon la revendication 2, **caractérisée en ce que** les peaux sensibles présentent les symptômes choisis parmi les irritations cutanées et/ou les dartres et/ou les érythèmes et/ou les sensations dysesthésiques et/ou les sensations d'échauffement et/ou les prurits de la peau et/ou des muqueuses.

4. Utilisation selon l'une quelconque des revendication 1 à 3, **caractérisée en ce que** la composition comprend de plus au moins un actif pharmaceutique, dermatologique ou cosmétique différent des sels.

5. Utilisation selon l'une quelconque des revendications 1 à 4, **caractérisée en ce que** le sel est constitué d'ions choisis parmi les ions chlorure, borate, bicarbonate, carbonate, nitrate, hydroxyde, sulfate, glycérophosphate, les acides de fruit, les acides aminés.

6. Utilisation selon la revendication précédente, **caractérisée en ce que** les ions sont les ions chlorure ou nitrate.

7. Utilisation selon l'une quelconque des revendications 1 à 6, **caractérisée en ce que** le sel est utilisé en une quantité allant de 10⁻⁵% à 20 % du poids total de la composition.

8. Utilisation selon l'une quelconque des revendications 1 à 7, **caractérisée en ce que** le sel est utilisé en une quantité représentant de 10⁻² % à 15 % du poids total de la composition et mieux de 0,5 à 8%.

9. Utilisation selon l'une quelconque des revendications 1 à 8, **caractérisé en ce que** la composition contient, en outre, au moins un agent choisi parmi les agents antibactériens, antiparasitaires, antifongiques, anti-inflammatoires, antiprurigineux, kératolytiques, anti-radicaux libres, anti-séborrhéiques, antipelliculaires, antiacnéiques et/ou les agents modulant la différenciation et/ou la prolifération et/ou la pigmentation cutanée.

10. Utilisation selon l'une quelconque des revendications 1 à 9, **caractérisée en ce que** la composition se présente sous forme d'une solution aqueuse, huileuse, hydroalcoolique, d'une émulsion huile-dans-eau ou eau-dans-huile, d'une microémulsion, d'un gel aqueux ou anhydre, d'un sérum, d'une dispersion de vésicules.

11. Utilisation selon l'une quelconque des revendications 1 à 10, **caractérisée en ce que** la composition contient, en outre, au moins un antagoniste choisi parmi les antagonistes de CGRP, d'histamine, d'interteukine-1 et/ou de TNFα, et de substance P, différent des dits sels.

12. Composition cosmétique, pharmaceutique ou dermatologique comprenant dans un milieu cosmétiquement, pharmaceutiquement ou dermatologiquement acceptable au moins un produit à effet secondaire irritant, **caractérisée en ce qu'**elle comprend, en outre, au moins un sel d'yttrium ou de néodyme, le dit produit à effet secondaire irritant étant choisi parmi les tensioactifs, les conservateurs, les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les teintures ou colorants capillaires, les agents antitranspirants, les actifs dépilatoires ou de permanentes, les actifs dépigmentants.

13. Composition selon la revendication précédente, **caractérisée en ce que** le sel est utilisé en une quantité représentant de 10⁻⁵ % à 20 % du poids total de la composition.

14. Composition selon l'une quelconque des revendications 12 ou 13, **caractérisée en ce que** le sel est utilisé en une quantité représentant de 10⁻² % à 15 % du poids total de la composition et mieux de 0,5 à 8%.

15. Composition selon l'une quelconque des revendications 12 à 14, **caractérisée en ce qu'**elle contient en outre, au moins un antagoniste choisi parmi les antagonistes de CGRP, d'histamine, d'interleukine-1 et/ou de TNFα, et de substance P, différent des dits sels d'yttrium ou de néodyme.

16. Composition selon la revendication précédente, **caractérisée en ce que** l'antagoniste de CGRP est un extrait d'lridacée.

## Patentansprüche

1. Verwendung mindestens eines Salzes, das unter den Yttriumsalzen, Lanthansalzen, Cersalzen, Praseodymsalzen, Promethiumsalzen, Samariumsalzen, Europiumsalzen, Gadoliniumsalzen, Terbiumsalzen, Dysprosiumsalzen, Holmiumsalzen, Erbiumsalzen, Thuliumsalzen, Ytterbiumsalzen, Lutetiumsalzen, Mangansalzen und deren Gemischen ausgewählt ist, zur Herstellung einer kosmetischen, dermatologischen oder pharmazeutischen Zusammensetzung, die zur Behandlung von Erkrankungen des Zentralnervensystems, Fibrosen, Störungen bei der Kollagenbildung, kardio-vaskulären Störungen, vasospastischen Störungen, immunologischen Störungen, Störungen des Harn- und Genitaltrakts und/oder pankreatischen Störungen vorgesehen ist.

2. Verwendung mindestens eines Salzes, das unter den Yttriumsalzen, Neodymsalzen, Promethiumsalzen, Samariumsalzen, Europiumsalzen, Terbiumsalzen, Dysprosiumsalzen, Holmiumsalzen, Erbiumsalzen, Thuliumsalzen, Ytterbiumsalzen und deren Gemischen ausgewählt ist, zur Herstellung einer kosmetischen oder dermatologischen Zusammensetzung, die für die Behandlung von empfindlicher Haut vorgesehen ist.

3. Verwendung nach Anspruch 2, **dadurch gekennzeichnet, dass** die empfindliche Haut Symptome aufweist, die unter Hautreizungen und/oder Flechten und/oder Erythemen und/oder dysästhesische Empfindungen und/oder Brennen und/oder Juckreiz der Haut und/oder der Schleimhäute ausgewählt sind.

4. Verwendung nach einem der Ansprüche 1 bis 3, **dadurch gekennzeichnet, dass** die Zusammensetzung ferner mindestens einen pharmazeutischen, dermatologischen oder kosmetischen Wirkstoff enthält, der von den Salzen verschieden ist.

5. Verwendung nach einem der Ansprüche 1 bis 4, **dadurch gekennzeichnet, dass** die Salze Ionen enthalten, die unter den Ionen Chlorid, Borat, Bicarbonat, Carbonat, Nitrat, Hydroxid, Sulfat und Glycerophosphat, Fruchtsäureionen und Aminosäureionen ausgewählt sind.

6. Verwendung nach dem vorhergehenden Anspruch, **dadurch gekennzeichnet, dass** es sich bei den Ionen um Chlorid und Nitrat handelt.

7. Verwendung nach einem der Ansprüche 1 bis 6, **dadurch gekennzeichnet, dass** das Salz in einem Anteil von 10⁻⁵ bis 20 % des Gesamtgewichts der Zusammensetzung verwendet wird.

8. Verwendung nach einem der Ansprüche 1 bis 7, **dadurch gekennzeichnet, dass** das Salz in einem Anteil von 10⁻² bis 15 % des Gesamtgewichts der Zusammensetzung und noch besser 0,5 bis 8 % verwendet wird.

9. Verwendung nach einem der Ansprüche 1 bis 8, **dadurch gekennzeichnet, dass** die Zusammensetzung ferner mindestens einen Wirkstoff enthält, der unter den antibakteriellen Wirkstoffen, antiparasitären Wirkstoffen, Antimykotika, entzündungshemmenden Wirkstoffen, juckreizstillenden Wirkstoffen, Keratolytika, Radikalfängern für freie Radikale, Antiseborrhoeika, Antischuppenmitteln, Wirkstoffen gegen Akne und/ oder Mitteln, die die Differenzierung und/oder Proliferation und/oder Pigmentierung der Haut modulieren, ausgewählt ist.

10. Verwendung nach einem der Ansprüche 1 bis 9, **dadurch gekennzeichnet, dass** die Zusammensetzung als wässrige, ölige oder wässrig-alkoholische Lösung, Öl-in-Wasser- oder Wasser-in-Öl-Emulsion, Mikroemulsion, wasserfreies oder wässriges Gel, Serum oder Vesikeldispersion vorliegt.

11. Verwendung nach einem der Ansprüche 1 bis 10, **dadurch gekennzeichnet, dass** die Zusammensetzung ferner mindestens einen Antagonisten enthält, der unter Antagonisten von CGRP, Histamin, Interleukin-1 und/oder TNF-α ausgewählt ist.

12. Kosmetische, pharmazeutische oder dermatologische Zusammensetzung, die in einem kosmetisch, pharmazeutisch oder dermatologisch akzeptablen Medium mindestens ein Produkt mit reizender Nebenwirkung enthält, **dadurch gekennzeichnet, dass** sie ferner mindestens ein Yttriumsalz oder Neodymsalz enthält, wobei das Produkt mit reizender Nebenwirkung unter den grenzflächenaktiven Stoffen, Konservierungsmitteln, α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Farbmitteln oder Farbstoffen für das Haar, Antitranspirantien, Haarentfernungsmitteln, permanent verformenden Stoffen oder depigmentierenden Mitteln ausgewählt ist.

13. Zusammensetzung nach dem vorhergehenden Anspruch, **dadurch gekennzeichnet, dass** das Salz in einem Anteil von 10⁻⁵ bis 20 % des Gesamtgewichts der Zusammensetzung verwendet wird.

14. Zusammensetzung nach Ansprüche 12 oder 13, **dadurch gekennzeichnet, dass** das Salz in einem Anteil von 10⁻² bis 15 % des Gesamtgewichts der Zusammensetzung und noch besser 0,5 bis 8 % verwendet wird.

15. Zusammensetzung nach einem der Ansprüche 12 bis 14, **dadurch gekennzeichnet, dass** sie ferner mindestens einen Antagonisten enthält, der unter den Antagonisten von CGRP, Histamin, Interleukin-1 und/ oder TNF-α und Antagonisten der Substanz P, die keine Yttriumsalze oder Neodymsalze sind, ausgewählt ist.

16. Zusammensetzung nach dem vorhergehenden Anspruch, **dadurch gekennzeichnet, dass** es sich bei dem CGRP-Extrakt um einen Extrakt aus Iridaceae handelt.

## Claims

1. Use of at least one salt chosen from yttrium, lanthanum, cerium, praseodymium, promethium, samarium, europium, gadolinium, terbium, dysprosium, holmium, erbium, thulium, ytterbium, lutetium or manganese salts and a mixture thereof, for the manufacture of a cosmetic, dermatological or pharmaceutical composition intended for treating central nervous system disorders, fibrosis, collagen maturation disorders, cardiovascular disorders, vasospastic disorders, immunological disorders and/or urogenital tract disorders and pancreatic disorders.

2. Use of at least one salt chosen from yttrium, neodymium, promethium, samarium, europium, terbium dysprosium, holmium, erbium, thulium, or ytterbium salts and a mixture thereof, for the manufacture of a cosmetic or dermatological composition intended for treating sensitive skins.

3. Use according to Claim 2, **characterized in that** the sensitive skins exhibit symptoms chosen from skin irritations and/or scurf and/or erythema and/or dysaesthesic sensations and/or sensations of chafing and/or pruritus of the skin and/or of the mucous membranes.

4. Use according to any one of Claims 1 to 3, **characterized in that** the composition further comprises at least one pharmaceutical, dermatological or cosmetic active agent different from the salts.

5. Use according to any one of Claims 1 to 4, **characterized in that** the salt consists of ions chosen from chloride, borate, bicarbonate, carbonate, nitrate, hydroxide, sulphate and glycerophosphate ions, fruit acids and amino acids.

6. Use according to the preceding claim, **characterized in that** the ions are chloride or nitrate ions.

7. Use according to any one of claims 1 to 6, **characterized in that** the salt is used in a quantity ranging from 10⁻⁵% to 20% of the total weight of the composition.

8. Use according to any one of claims 1 to 7, **characterized in that** the salt is used in a quantity representing from 10⁻²% to 15% of the total weight of the composition, and even better from 0.5 to 8%.

9. Use according to any one of claims 1 to 8, **characterized in that** the composition additionally contains at least one agent chosen from antibacterial, antiparasitic, antifungal, anti-inflammatory, anti-pruritic, keratolytic, anti-free radical; antiseborrhoeic, antidandruff, anti-acne agents and/or skin differentiation and/or proliferation and/or pigmentation modulating agents.

10. Use according to any one of claims 1 to 9, **characterized in that** the composition is provided in the form of an aqueous, oily or aqueous-alcoholic solution, an oil-in-water or water-in-oil emulsion, a microemulsion, an aqueous or anhydrous gel, a serum, or a dispersion of vesicles.

11. Use according to any one of Claims 1 to 10, **characterized in that** the composition additionally contains at least one antagonist chosen from antagonists of CGRP, of histamine, of interleukin-1 and/or of TNFα, and of substance P, different from the said salts.

12. Cosmetic, pharmaceutical or dermatological composition comprising, in a cosmetically, pharmaceutically or dermatologically acceptable medium, at least one product with an irritant side effect, **characterized in that** it additionally comprises at least one yttrium or neodymium salt, the said product with an irritant side effect being chosen from surfactants, preservatives, α-hydroxy acids, β-hydroxy acids, α-keto acids, β-keto acids, retinoids, anthralins, anthranoids, peroxides, minoxidil, lithium salts, antimetabolites, vitamin D and its derivatives, hair dyes or colorants, antiperspirants, depilatory or permanent waving active agents and depigmenting active agents.

13. Composition according to the preceding claim, **characterized in that** the salt is used in a quantity representing from 10⁻⁵% to 20% of the total weight of the composition.

14. Composition according to claims 12 or 13, **characterized in that** the salt is used in a quantity representing from 10⁻²% to 15% of the total weight of the composition, and even better from 0.5 to 8%.

15. Composition according to any one of claims 12 to 14, **characterized in that** it additionally contains at least one antagonist chosen from antagonists of CGRP, of histamine, of interleukin-1 and/or of TNFα, and of substance P, different from the said yttrium or neodymium salts.

16. Composition according to the preceding claim, **characterized in that** the antagonist of CGRP is an extract of Iridaceae.
